(19) Europäisches Patentamt
European Patent Office
Office européen des brevets

(11) Numéro de publication: **0 176 412 B1**

(12) **FASCICULE DE BREVET EUROPEEN**

(45) Date de publication du fascicule du brevet: **17.11.88**

(51) Int. Cl.⁴: **C 07 C 69/96**, C 07 C 68/06

(21) Numéro de dépôt: **85401723.3**

(22) Date de dépôt: **05.09.85**

(54) **Nouveau procédé de préparation de fluoroformiates.**

(30) Priorité: **17.09.84 US 651661**

(43) Date de publication de la demande: **02.04.86 Bulletin 86/14**

(45) Mention de la délivrance du brevet: **17.11.88 Bulletin 88/46**

(84) Etats contractants désignés: **BE CH DE FR GB IT LI NL SE**

(56) Documents cités: **Néant**

(73) Titulaire: **SOCIETE NATIONALE DES POUDRES ET EXPLOSIFS, 12, quai Henri IV, F-75181 Paris Cédex 04 (FR)**

(72) Inventeur: **Piteau, Marc, 38, avenue de Ballancourt, F-91710 Itteville (FR)**
Inventeur: **Senet, Jean-Pierre, 79 rue de Gare Herbeauvilliers-Buthiers, F-77760 La Chapelle-la-Reine (FR)**
Inventeur: **Wolf, Patrick, 17, avenue du Maréchal Joffre, F-91710 Vert-le-Petit (FR)**
Inventeur: **Dang, Vu-Anh, 331 W. College Aveue Room 300, State College Pennsylvania 16803 (US)**
Inventeur: **Olofson, Roy Arne, Prof. PENNSTATE UNIVERSITY, Col. Science Dep. Chemistry 152 Davey Laboratory, University Park Pennsylvania 16803 (US)**

EP 0 176 412 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de fluoroformiates de formule:

$$R^1-O-\underset{\underset{O}{\|}}{C}-F$$

Les fluoroformiates sont des composés connus et recherchés, notamment pour introduire des groupes oxycarbonylés dans une molécule, par exemple en synthèse peptidique.

Quelques procédés de préparation ont déjà été proposés mais ils ne donnent pas entière satisfaction.

Un de ces procédés consiste à faire réagir un fluorohalogénure de carbonyle avec un alcool ou un phénol (J. Org. Chem. (1956) 21 p. 1 319) selon la réaction:

$$ROH + X-\underset{\underset{O}{\|}}{C}-F \longrightarrow R-O-\underset{\underset{O}{\|}}{C}-F + HX$$

X représente F, Cl ou Br

Mais ce procédé présente plusieurs inconvénients. Les halogénures de fluorocarbonyle sont très difficiles à préparer et par conséquent très peu courants. Ils sont également difficiles à manipuler et dangereux. La température de la réaction doit être très basse, aux environs de −70 °C et un cycle complexe de température de −70 °C à 0 °C est mis en œuvre ce qui entraîne des frais opératoires très élevés. Le fluoroformiate obtenu est impur en raison des sous-produits formés tels que des carbonates ou des produits de départ non transformés. Les rendements sont faibles 50 à 55%. Si l'on veut les améliorer, il faut utiliser des pressions élevées (Brevet FR N° 2 010 922), ce qui n'est pas sans risques.

Une autre préparation plus courante s'effectue à partir de chloroformiates par échange chlore-fluor au moyen d'un fluorure mais les conditions de la réaction, haute température ou présence de catalyseurs ne permettent pas toujours d'obtenir le fluoroformiate désiré, notamment lorsque le chloroformiate de départ est très instable. Celui de tertiobutyle par exemple se décompose rapidement et violemment en libérant de l'isobutène, du gaz carbonique et de l'acide chlorhydrique (J. Amer. Chem. Soc (1957) 79 – p. 4 686). Sa formation in situ ne donne pas de meilleur résultat (J. Amer. Chem. Soc. (1966) 88 p. 852). A partir du chloroformiate de benzyle on obtient en majorité du chlorure de benzyle. La substitution du chlore par le fluor devient de plus en plus difficile lorsque le nombre de carbone augmente dans les chloroformiates primaires ou secondaires.

La mise au point d'un nouveau procédé général de préparation en grandes quantités, sans risque important et à un coût minimal, de nombreux fluoroformiates, d'une grande pureté, et en particulier des plus inaccessibles d'entre eux était donc particulièrement souhaitable.

L'invention consiste à préparer les fluoroformiates de formule

$$R^1-O-\underset{\underset{O}{\|}}{C}-F,$$

dans laquelle $R^1$ représente un reste aliphatique, cycloaliphatique ou polycyclique, substitué ou non, saturé ou non, un reste araliphatique substitué ou non ou un reste aromatique, en faisant réagir un carbonate de formule

$$R^1-O-\underset{\underset{O}{\|}}{C}-O-\underset{\underset{Cl}{|}}{CH}-R^2$$

dans laquelle $R^1$ a la signification précédente et $R^2$ représente un atome d'hydrogène, un reste alkyle en $C_1$ à $C_{12}$ ou cycloalkyle en $C_5$à $C_{12}$, saturé ou non, substitué ou non par un ou plusieurs atomes d'halogène ou un reste aryle substitué ou non par un ou plusieurs atomes d'halogène, avec un fluorure alcalin ou alcalino-terreux, d'ammonium ou d'ammonium quaternaire $NR^3R^4R^5R^6$ dans lequel $R^3$, $R^4$, $R^5$, $R^6$ identiques ou différents représentent un atome d'hydrogène ou un groupe alkyle ou aralkyle en $C_1$ à $C_{12}$ ou un fluorure minéral choisi parmi $KHF_2$, $NH_4HF_2$ ou $KSO_2F$. Le fluorure est activé soit par un agent complexant du cation choisi parmi les cryptates et les polyéthers cycliques ou linéaires, soit par un milieu aprotique polaire. La température de réaction est comprise entre 20° et 120 °C. Le fluoroformiate ou l'aldéhyde obtenu ou les deux sont séparés du milieu réactionnel au fur et à mesure de leur formation.

Le schéma réactionnel peut s'écrire:

$$n(R^1-O-\underset{\underset{O}{\|}}{C}-O-\underset{\underset{Cl}{|}}{CH}-R^2) + MF_n \rightarrow nR^1-O-\underset{\underset{O}{\|}}{C}-F + nR^2CHO + MCl_n$$

n est égal à 1 ou 2.

La réaction peut être effectuée lorsqu'on utilise un agent complexant en présence ou non d'un solvant inerte vis-à-vis des réactifs.

Les carbonates α-chlorés de départ sont disponibles dans le commerce ou sont préparés facilement par des méthodes connues en soi, par exemple par condensation des chloroformiates α-chlorés avec des dérivés hydroxylés (Chem. Rev. 1964 pp. 654–687).

La radical $R^1$ peut être très varié. Généralement, il comporte de 1 à 40, de préférence de 1 à 24 atomes de carbone et ses substituants sont choisis parmi les radicaux hydrocarbonés et les atomes d'halogène. A titre d'exemples on peut citer comme reste

– alkyle: $CH_3$, $C_2H_5$, n–$C_3H_7$, i–$C_3H_7$, allyle, n–

$C_4H_9$, t–$C_4H_9$, t–$C_5H_{11}$, néopentyle, n–$C_8H_{17}$, octadécyle,

- cycloalkyle et polycyclique: cyclohexyle, 1-adamantyle, cholestéryle,
- aryle: phényle, naphtyle,
- aralkyle: benzyle, halogénobenzyle.

Les radicaux aliphatiques ou cycloaliphatiques tertiaires sont appréciés, en particulier le radical tertiobutyle.

Dans un but de simplidication la définition précédente de $R^1$ a été donnée en considérant qu'il n'est rattaché qu'à une seule fonction carbonate. Il est bien certain que dans le cadre de l'invention $R^1$ pourrait être rattachée à une ou plusieurs autres fonctions carbonates α-chlorés et que le procédé de l'invention permet d'obtenir des polyfluoroformiates, par exemple des bisfluoroformiates d'alkylèneglycol à partir de biscarbonates d'alkylèneglycol.

Le radical $R^2$ se retrouve dans l'aldéhyde formé comme produit secondaire. Ce peut être un radical très varié. Le plus souvent il sera choisi en fonction de la facilité de formation ou d'élimination de l'aldéhyde ou pour des raisons économiques.

On peut citer à titre d'exemples pour $R^2$: $CH_3$, $C_4H_9$, $CCl_3$, $CH_2 = CH–$, cyclohexyle, phényle, chloro-1-phényle.

Le radical $CCl_3$ est particulièrement apprécié. Le radical vinyle convient bien également. Lorsque $R^2$ est un méthyle, les carbonates sont plus accessibles et l'acétaldéhyde s'élimine plus facilement du milieu réactionnel.

Selon l'invention on a trouvé que de façon surprenante les fluorures précédemment cités permettaient la coupure des carbonates α-chlorés et leur transformation en fluoroformiates. On les introduit le plus souvent en quantité comprise entre 1 et 5 équivalents, de préférence 1, 2 et 3 équivalents par rapport au carbonate α-chloré. Un grand excès de fluorure n'améliore pas nettement les rendements du procédé. La fluorure de potassium est généralement celui que l'on préfère utiliser.

Les fluorures doivent être nécessairement activés. Les cryptates ou les éthers-couronne qui complexent le cation, remplissent très bien cette fonction. Ils sont décrits dans l'article de Kappenstein «Bull. Soc. Chim. de France» 1974 n° 1–2 pages 89–109 ou dans celui de Lehn «Structure and Bonding», volume 16, page 2–64, Springer Verlag (1974). Les monoalkyléthers de polyéthylèneglycol de masse moléculaire comprise entre 500 et 10 000 peuvent également être utilisés. On peut citer par exemple le monométhyléther de masse moléculaire moyenne en poids égale à 5000.

Un agent complexant convenant bien, en particulier avec le fluorure de potassium est un cryptate choisi parmi les polyoxydiazamacrobicycles commercialisé par la Société Merck sous la marque déposée Kryptofix par exemple l'hexaoxa-4,7,13,16,21,24 diaza-1,10 bicyclo [8,8,8] hexacosane ou «Kryptofix 222» ou encore un éther-couronne tel que le 18-couronne-6 ou 1,4,7,10,13,16 hexaoxa cyclodécane. On introduit en général de 1 à 10% en mole de cet agent par rapport au fluorure.

On a remarqué que les fluorures sont également activés lorsqu'ils se trouvent dans un milieu aprotique polaire. Des milieux anhydres tels que le diméthylsulfoxyde, l'hexaméthylphosphorotriamide, la N-méthylpyrrolidone, le sulfolane et de préférence le diméthylformamide conviennent bien.

Pour améliorer les conditions réactionnelles on peut, lorsqu'on met en œuvre le fluorure sous forme complexée, utiliser un ou plusieurs solvants anhydres, inertes vis-à-vis du carbonate α-chloré. Les diéthers alkyliques d'alkylèneglycol ou de polyoxylkylèneglycol par exemple les glymes conviennent bien. D'autres solvants tel que le dioxanne et le benzonitrile peuvent également être utilisés.

Les réactions sont effectuées de préférence à une température comprise entre 10° et 70 °C.

Lorsque l'on prépare des fluoroformiates instables il faut évidemment ne pas choisir une température trop élevée. Dans le cas du fluoroformiate de tertiobutyle par exemple elle doit être inférieure à 55 °C.

Pour séparer le fluoroformiate et l'aldéhyde formés ou l'un des deux du milieu réactionnel, tout moyen connu de l'homme de l'art pourra être utilisé. Une méthode qui sera souvent employée consiste à évaporer en continu sous pression réduite les deux composés et à les séparer soit par un moyen physique, tel qu'une distillation fractionnée, soit par un moyen chimique. Les vapeurs de l'aldéhyde issues du mélange réactionnel peuvent par exemple être envoyées dans un alcool ou un polyol pour être transformées en acétal.

Lorsque le fluoroformiate est le composé le plus volatil il est extrait en continu du milieu réactionnel et on peut le purifier par distillation. Lorsqu'il reste dans le milieu, on peut effectuer les opérations courantes de lavage, par exemple avec du chloroforme et de l'eau glacée, de séchage et on l'obtient par distillation sous pression réduite.

Le procédé de l'invention permet à partir de matières premières courantes de préparer un grand nombre de fluoroformiates d'une grande pureté et avec un excellent rendement et en particulier des fluoroformiates dont la synthèse jusqu'à présent était difficile ou impossible.

Le procédé de l'invention, convient particulièrement bien pour la préparation du fluoroformiate de tertiobutyle qui posait de nombreux problèmes selon les méthodes antérieures.

La plupart des fluoroformiates obtenus présente une stabilité suffisante toutefois il peut être souhaitable pour certains tel que par exemple le fluoroformiate de tertiobutyle d'améliorer cette propriété. On a trouvé que l'addition au fluoroformiate après purification d'un composé tel qu'un carbonate alcalin ou alcalino-terreux a un effet tout à fait favorable.

On l'ajoute dans le fluoroformiate à raison de 1 à 25%, généralement 5 à 10% en poids par rapport à celui-ci. Un composé comme le carbonate de sodium anhydre convient bien.

Les fluoroformiates ont de nombreuses applications. Ils sont utiles pour former les fluorures d'alkyle (Brevet FR N° 1 549 815 Col. 1) ou d'aryle (CA-66-P 18 571). Ils sont également très précieux au cours d'opérations de synthèse organique, par exemple pour introduire des groupes protecteurs des fonctions amines de divers composés polyfonctionnels notamment des aminoacides dans la chimie des peptides (Brevet US N° 3 592 836), Houben Weyl Methoden der organischen Chemie, 15 Teil 1 Synthese von Peptiden, pp. 46–314) et comme intermédiaires pour la préparation de bactéricides et fongicides (CA-76-P. 24 899).

Les exemples suivants illustrent l'invention.

Exemple 1

Préparation du fluoroformiate de t-butyle

a) préparation du carbonate de t-butyle et de tétrachloro-1,2,2,2 éthyle

$(CH_3)_3$ C–O–C–O–CH–$CCl_3$
‖ |
O Cl

Dans un réacteur de 4 litres, on introduit 1 l de dichlorométhane, 155 g (2,1 moles) de t-butanol et 493 g (2 moles) du chloroformiate de tétrachloro-1,2,2,2 éthyle.

Sous agitation, à une température comprise entre 0° et 5 °C, on ajoute ensuite en 1 heure environ 160 g (2,025 moles) de pyridine en solution dans 400 ml de dichlorométhane. Après 4 heures d'agitation à 5 °C, le précipité formé est éliminé après lavage par 600 ml de dichlorométhane.

Les solutions de dichlorométhane sont lavées à l'eau jusqu'à neutralité, séchées sur sulfate de magnésium, puis le solvant est éliminé sous pression réduite. On isole ainsi 518 g (rendement 92%) du produit attendu sous forme de cristaux blancs, qui peut être purifié par distillation sous pression réduite (Point d'Ebullition, PE = 96 °C sous 900 Pa). Son point de fusion, PF est de 70 °C.

IR νC = 0:1770 $cm^{-1}$

RMN$^1$H ($CDCl_3$, TMS, δ ppm): 1,5 (s, $CH_3$) 6,7 (s, CH)

b) préparation du fluoroformiate de t-butyle

Dans un réacteur de 2 l équipé d'un agitateur, d'un thermomètre, d'un réfrigérant à reflux droit, refroidi par de l'eau à température courante, relié à un tube plongeant dans un second réacteur, on introduit 100 g (1,7 moles) de fluorure de potassium, 10 g (0,038 mole) de 18-couronne-6 puis une solution dans 300 ml de diméthyléther du diéthylèneglycol de 300 g (1,06 moles) du carbonate obtenu en a).

Dans le second réacteur muni d'un réfrigérant à reflux à chicanes et refroidi par de l'eau, on introduit 250 ml de diméthyléther de diéthylèneglycol, 70 g d'éthylèneglycol et 60 g de sulfate de magnésium anhydre, et l'ensemble est maintenu à 30–35 °C sous agitation.

Le mélange réactionnel du premier réacteur est maintenu à 50 °C sous agitation. L'ensemble des deux réacteurs est sous une pression de 1,3 kPa environ. On laisse la réaction se poursuivre 8 heures durant lesquelles il y a distillation continue du fluoroformiate de t-butyle et du chloral formé hors du premier réacteur vers le second où l'aldéhyde réagit avec l'éthylèneglycol donnant un acétal peu volatil. Seul la fluoroformiate distille hors du second réacteur et il se condense selon une technique classique, par exemple dans un piège maintenu à −50 °C et relié à une pompe à vide.

Après distillation sous pression réduite du produit ainsi isolé, on obtient 100 g (rendement 79%) de fluoroformiate de t-butyle, liquide distillant vers 6–8 °C sous 3,6 kPa (27 mm Hg).

IR νC = 0:1830 $cm^{-1}$

RMN$^1$H ($CDCl_3$, TMS, δ ppm): 1,54 d; $J_{H,F}$ = 1,47 Hz $(CH_3)_3$ C $^{19}$F (acide trifluoroacétique, ppm): 65,5 (s)

$N_D^{15}$: 1,3615

$d_4^{20}$: 0,987

Exemple 2

Préparation du fluoroformiate de t-butyle

Dans un réacteur semblable à celui de l'exemple 1 b on introduit 389 g (1,37 moles) de carbonate de t-butyle et tétrachloro-1,2,2,2 éthyle, 115 g (1,98 moles) de fluorure de potassium dans 700 ml de diméthylformamide anhydre, le réfrigérant est relié à un tube plongeant dans un second réacteur surmonté d'une colonne à distillation, garnie d'anneaux de Raschig, et comportant une double enveloppe, ce qui permet de la maintenir à 15 °C; la colonne est reliée à un piège maintenu à −70 °C lui-même relié à une pompe à vide. L'ensemble des réacteurs et piège est maintenu à une pression d'environ 2,7 kPa pendant toute la durée de la réaction.

Dans le second reacteur ont été préalablement introduits 140 g (2,25 moles) d'éthylèneglycol et 93 g (0,65 mole) de sulfate de sodium anhydre et l'ensemble est maintenu à 30–35 °C, sous agitation.

Le premier réacteur est maintenu à 50 °C pendant 8 heures, au cours desquelles le fluoroformiate formé se condense dans le piège à −70 °C. On obtient 123,7 g (rendement 75%) de fluoroformiate de tertiobutyle pur possédant les caractéristiques indiquées à l'exemple 1 b.

Exemple 3

Préparation du fluoroformiate de t-butyle

Dans le réacteur équipé d'un agiteur, d'un thermomètre et d'un réfrigérant à reflux, on introduit 33,3 g (0,2 mole) de carbonate de t-butyle et de chlorométhyle, 15,1 g (0,26 mole) de fluorure de potassium, 4,74 g (0,018 mole) de 18-couronne-6 et 60 ml de dioxanne. On agite le mélange pendant 24 h à 50 °C. On constate par analyse RMN du milieu réactionnel que l'on obtient le fluoroformiate de t-butyle avec un rendement de 40% environ.

Exemple 4

Préparation du fluoroformiate de phényle

On mélange 11,6 g (0,058 mole) de carbonate de chloro-1-éthyle et de phényle fraîchement distillé, 4,60 g (0,079 mole) de KF anhydre (Al-

drich), 0,80 g (0,003 mole) de 18-couronne-6 (Aldrich). On agite et on chauffe le mélange au moyen d'un bain d'huile à 75 °C, sous une pression de 2,7 kPa environ. On laisse la réaction se poursuivre 90 minutes pendant lesquelles il y a évaporation et distillation fractionnée en continu du fluoroformiate de phényle formé.

On recueille 5,67 g de fluoroformiate de phényle soit un rendement de 70%.

PE: 60–63 °C à 2,7 kPa

IR ($CCl_4$): 5,42 μ ( >C = 0), 6,29 μ (m)

RMN$^1$H ($CCl_4$, δ): 7,25 (large s)

Exemple 5

Préparation du fluoroformiate de t-butyle

On ajoute à 3 g (0,0516 mole) de KF anhydre et 0,37 g (0,001 mole) de 18-couronne-6, 5 g (0,03 mole) de carbonate de t-butyle et de chloro-1-éthyle fraîchement distillé.

On agite et on chauffe mélange à environ 70 °C (bain d'huile) sous une pression de 4,9 kPa et on condense dans un piège à −80 °C les produits qui se forment.

En 18 heures on recueille 3,73 g d'un mélange d'aldéhyde et de fluoroformiate. On chauffe à 50 °C pour éliminer l'acétaldéhyde puis on distille sous pression réduite pour isoler le fluoroformiate pur (PE: 45 °C sous 27 kPa). On en obtient 2,66 g (rendement 80%). Ses caractéristiques IR et RMN sont les mêmes que celles indiquées à l'exemple 1b.

Exemple 6

Préparation du fluoroformiate d'éthyle

Dans un réacteur relié à un piège à −80°, on effectue un mélange de 6 g (0,04 mole) de carbonate de chloro-1-éthyle et d'éthyle fraîchement distillé, de 10 g (0,17 mole) de KF anhydre, de 2,4 g (0,01 mole) de 18-couronne-6 dans 17 ml de benzonitrile (Aldrich, séché sur $CaCl_2$ et distillé sous vide en présence de $P_2O_5$) que l'on agite et que l'on chauffe à 55 °C (bain d'huile) sous une pression de 4 kPa. Après 13 heures on recueille dans le piège 5 g d'un mélange d'acétaldéhyde et de fluoroformiate d'éthyle. Le rendement de ce dernier par analyse RMN$^1$H est de 93%.

IR ($CCl_4$): 5,46 μ, 8,01

RMN$^1$H ($CCl_4$, δ ppm): 4,50 (q, 2H, J=7Hz) 1,44 (d de t, 3H, J=7 et 2 Hz)

Exemple 7

Préparation du fluoroformiate de n-octyle

On agite et on chauffe à environ 85 °C (bain d'huile) sous une pression d'environ 1,87 kPa, un mélange de 10,6 g (0,045 mole) de carbonate d'octyle et de chloro-1 éthyle distillé au préalable, de 4,5 g (0,08 mole) de KF sec et de 0,6 g (0,002 mole) de 18-couronne-6. Après 12 heures, tout l'acétaldéhyde volatile s'étant échappé, on refroidit le mélange et on le distille sous pression réduite. On obtient 6,87 g de fluoroformiate de n-octyle pur (rendement 86%)

PE: 74–77 °C à 533 Pa (4 mm Hg)

IR ($CCl_4$): 5,46 μ

RMN$^1$H ($CCl_4$, δ): 4,20 (t, 2H, J=6Hz), 0,8–1,6 (m, 15H)

Exemple 8

Préparation du fluoroformiate de t-amyle

On agite et on chauffe à environ 70 °C (bain d'huile) sous une pression de 1,87 kPa, une bouillie de 10,4 g (0,054 mole) de carbonate de chloro-1-éthyle et de t-amyle, de 4,5 g (0,077 mole) de KF sec et de 0,67 g de 18-couronne-6 (0,0025 moles). On condense les composés volatils dans un piège à −80 °C dès qu'ils se forment. Au bout de 34 heures on obtient 8,18 g d'un mélange d'aldéhyde et de fluoroformiate de t-amyle. Par distillation sous pression réduite on obtient 5,99 g (rendement 83%) de fluoroformiate pur dont le point d'ébullition est à 61–65 °C sous 17,3 kPa

IR ($CCl_4$): 5,46 μ (s)

RMN$^1$H ($CDCl_3$, δ): 1,85 (q, 2H, J=7Hz), 1,50 (d, j=1Hz, 6H), 0,93 (t, J=7Hz, 3H)

Exemple 9

Préparation du fluoroformiate d'adamantyle-1

On agite et on chauffe à environ 120 °C (bain d'huile) sous une pression de 160 Pa une bouillie de 9,93 g (0,038 mole) de carbonate de chloro-1-éthyle et d'adamantyle-1, de 3,4 g (0,06 mole) de KF anhydre et de 0,43 g (0,0016 mole) de 18-couronne-6. On distille en continu l'acétaldéhyde et le fluoroformiate et on empêche ce dernier de se solidifier dans le réfrigérant au moyen d'un courant d'air chaud extérieur. Au bout de 36 heures on obtient 6,17 g de fluoroformiate pratiquement pur (environ 1% de 18-couronne-6 sont détectés par analyse RMN$^1$H) rendement 81%. On distille et on obtient 5,79 g du composé pur (rendement 76%) qui se solidifie dans la recette.

PE: 71–75 °C sous 160 Pa

PF: 30–32 °C

IR ($CCl_4$): 5,47 μ,

RMN$^1$H ($CCl_4$, δ): 2,18 (centre de la bande large s, 9H), 1,82 (centre de la bande s, 6H).

Exemple 10

Préparation du fluoroformiate de benzyle

On agit et on chauffe à environ 55 °C (bain d'huile) sous une pression de 160 Pa un mélange de 8,01 g (0,037 mole) de carbonate de chloro-1-éthyle et de benzyle, de 3,89 g (0,067 mole) de KF et de 0,49 g (0,002 mole) de 18-couronne-6. On évapore au fur et à mesure le fluoroformiate qui est condensé dans un piège froid et l'acétaldéhyde qui s'échappe. Après 4 heures on termine l'évaporation à l'aide d'un courant d'air chaud extérieur. On obtient un mélange de fluoroformiate et de carbonate de départ que l'on distille pour isoler 3,45 g (rendement 60%) de fluoroformiate de benzyle pur.

PE: 44–46 °C à 160 Pa

IR ($CCl_4$): 5,45 μ

RMN$^1$H ($CCl_4$, δ): 7,42 (s, 5H), 5,25 (s, 2H)

Exemples 11 et 12

On prépare les fluoroformiates selon le mode

opératoire suivant: on agite et on chauffe à la température de 50–60 °C, le carbonate (1 éq), KF (1,6 éq), le 18-couronne-6 (0,06 éq) dans du tétraglyme sous une pression d'environ 1,33 à 2,7 kPa. Les volatils sont évaporés en continu et recueillis dans un piège à −70 °C. Le fluoroformiate est ensuite isolé par distillation.

Préparation du fluoroformiate d'isopropyle

A partir du carbonate de (chloro-2) phényle-1-chloro méthyle et d'isopropyle (PE: 100° sous 27 Pa)

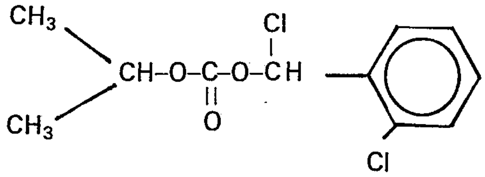

et après 13 heures de réaction on obtient le fluoroformiate d'isopropyle avec un rendement de 70% par analyse RMN$^1$H.

IR νC = 0:1830 cm$^{-1}$ large bande C–O–C à 1275 cm$^{-1}$

RMN$^1$H (δ ppm): 1,4 (doublet «dédoublé», 6H), 4,95 (m, 1H)

Préparation du fluoroformiate de cyclohexyle

A partir du carbonate de cyclohexyle et de chloro-1-pentyle (PE: 90 °C sous 20 Pa) on obtient un mélange de fluoroformiate de cyclohexyle (rendement 20% par analyse RMN$^1$H) et de valéraldéhyde. On isole le fluoroformiate par distillation à 55°–56 °C sous 2,9 kPa.

IR νC = 0:1830 cm$^{-1}$, large bande à 1275 cm$^{-1}$

RMN$^1$H ($CDCl_3$, δ ppm): 4,70 (m, 1H), 1,55 (m, 10H)

Exemple 13

Préparation du fluoroformiate de n-octadécyle

On agite et on chauffe à environ 90 °C (bain d'huile), sous une pression de 666 Pa, pendant 30 heures, un mélange de 4,65 g (0,012 moles) de carbonate de chloro-1-éthyle et d'octadécyle, de 1,39 g de KF anhydre (0,0239 mole) et de 0,25 g (0,95 mmole) de 18-couronne-6. Puis on refroidit le mélange restant, on le dilue avec $CHCl_3$ (20 ml), on le lave rapidement avec de l'eau glacée (3 × 15 ml) et on le sèche ($Na_2SO_4$). On distille sous vide et on obtient 2,82 g de fluoroformiate pur (rendement 72%), point d'ébullition 175–180 °C à 3,33 kPa

IR ($CCl_4$): 5,46 μ

RMN$^1$H ($CCl_4$, δ): 4,25 (t, 2H, J=6Hz), 1,3 (large s, 32H), 0,90 (t, 3H, J=6Hz)

Exemple 14

Préparation du fluoroformiate de néopentyle

1) A partir de carbonate de néopentyle et de chloro-1-éthyle.

On agite et on chauffe à environ 60 °C (bain d'huile) sous une pression réduite de 2,9 kPa, un mélange de 5,50 g (0,028 mole) de carbonate de néopentyle et de chloro-1-éthyle, de 2,5 g de KF anhydre (0,043 mole), de 14 g de monométhyléther de polyéthylèneglycol (Mp moyen = 5000, Aldrich) et de 15 ml de benzonitrile. On condense les composés produits dans un piège à (−80 °C) au fur et à mesure qu'ils se forment. On recueille 2,71 g d'aldéhyde et de fluoroformiate au bout de 3,5 jours. Par distillation à 55–57 °C sous 16 kPa on obtient 1,97 g de fluoroformiate pur (rendement 52%)

IR ($CCl_4$): 5,46 μ

RMN$^1$H ($CCl_4$, δ): 3,98 (s, 2H), 0,98 (s, 9H).

2) A partir du carbonate de néopentyle et de tétrachloro-1,2,2,2 éthyle.

On agite et on chauffe à 65 °C (bain d'huile) un mélange de 3,3 g (0,011 mole) de carbonate de néopentyle et de tétrachloro-1,2,2,2 éthyle, de 1 g de KF anhydre (0,017 mole), de 5 g (0,001 mole) de monométhyléther de polyéthylèneglycol (Mp moyen = 5000, Aldrich) et 10 ml de benzonitrile. On arrête la réaction au bout de 34 heures. Par analyse IR et RMN on détermine que le fluoroformiate est obtenu avec un rendement de 76%.

Exemple 15

Préparation de fluoroformiate de cholestéryle

On agite et on chauffe à environ 40 °C (bain d'huile) sous une pression de 399 Pa un mélange de 1 g (0,002 mole) de carbonate de cholestéryle et de chloro-1-éthyle, 0,25 g de KF anhydre (0,004 mole) et 0,05 g (0,2 mmole) de 18-couronne-6 dans 2 ml de benzonitrile. Après 31 heures on refroidit le mélange jusqu'à la température ambiante on le dilue avec $CH_2Cl_2$, on le filtre, on le concentre et on cristallise le résidu au moyen d'acétonitrile sec. On obtient 0,72 g (rendement 82%) de fluoroformiate.

PF: 114–117 °C

IR ($CCl_4$): 5,48, 8,03 μ

RMN$^1$H ($CCl_4$, δ): 5,2–5,5 (m), 4,2–4,6 (m) 0,7–2,6 (m)

Exemple 16

Préparation du fluoroformiate de t-butyle

On chauffe à 70° sous une pression de 4,7–5,3 kPa un mélange de 9 g (0,047 mole) de carbonate de t-butyle et d'α-chloroallyle, de 3,6 g de KF (0,061 mole) et 0,7 g (0,003 mole) de 18-couronne-6. On recueille le fluoroformiate et l'acroleine dans un piège à −80 °C. On arrête la réaction au bout de 10 heures. Par analyse RMN$^1$H on détermine qu'il reste 29% des produits de départ dans le réacteur. On sépare le fluoroformiate de t-butyle de l'acroléine par destillation fractionnée: PE = 45–45 °C sous 26 kPa. On en recueille 3,2 g (rendement 55%).

**Revendications**

1. Procédé de préparation de fluoroformiates de formule

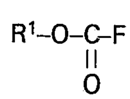

dans laquelle $R^1$ représente un reste aliphatique, cycloaliphatique ou polycyclique substitué ou non, saturé ou non, un reste araliphatique substitué ou non ou un reste aromatique, caractérisé en ce qu'on fait réagir un carbonate de formule

$$R^1\text{–O–}\underset{\underset{\displaystyle O}{\|}}{C}\text{–O–}\underset{\underset{\displaystyle Cl}{|}}{CH}\text{–}R^2$$

dans laquelle $R^1$ a la signification précédente et $R^2$ représente un atome d'hydrogène, un reste alkyle en $C_1$ à $C_{12}$, cycloalkyle en $C_5$ à $C_{12}$, saturé ou non, substitué ou non par un ou plusieurs atomes d'halogène ou un reste aryle substitué ou non par un ou plusieurs atomes d'halogène, avec un fluorure alcalin ou alcalino-terreux, d'ammonium ou d'ammonium quaternaire $NR^3R^4R^5R^6$ dans lequel $R^3$, $R^4$, $R^5$ et $R^6$ identiques ou différents représentent un atome d'hydrogène ou un groupe alkyle ou aralkyle en $C_1$ à $C_{12}$ ou avec un fluorure minéral choisi parmi $KHF_2$, $NH_4HF_2$ ou $KSO_2F$, le fluorure étant activé soit par un agent complexant du cation choisi parmi les cryptates, les polyéthers cycliques ou linéaires, soit par un milieu aprotique polaire, à une température de réaction comprise entre 20 et 120 °C, qu'on sépare du milieu réactionnel le fluoroformiate ou l'aldéhyde obtenu, ou les deux, au fur et à mesure de leur formation et qu'on récupère le fluoroformiate.

2. Procédé selon la revendication 1, caractérisé en ce que $R^1$ représente un groupe aliphatique ou cycloaliphatique tertiaire, un groupe polycycloaliphatique ou un group benzyle.

3. Procédé selon la revendication précédente caractérisé en ce que $R^1$ représente le groupe t-butyle, t-amyle, 1-adamantyle.

4. Procédé selon l'une quelconque des revendications précédentes caractérisé en ce que $R^2$ est le radical $-CCl_3$.

5. Procédé selon l'une quelconque des revendications précédentes caractérisé en ce que le milieu aprotique polaire est choisi parmi le diméthylformamide, le diméthylsulfoxyde, l'hexaméthylphosphorotriamide, la N-méthylpyrrolidone, le sulfolane.

6. Procédé selon l'une quelconque des revendications précédentes caractérisé en ce que le fluorure est utilisé en quantité comprise entre 1 et 5 équivalents par rapport au carbonate, de préférence 1, 2 et 3 équivalents.

7. Procédé selon l'une quelconque des revendications précédentes caractérisé en ce que l'agent complexant est ajouté à raison de 1 à 10% en mole par rapport au fluorure.

8. Procédé selon l'une quelconque des revendications précédentes caractérisé en ce que le fluorure est le fluorure de potassium.

9. Procédé selon la revendication précédente caractérisé en ce que le fluorure de potassium est complexé par le 18-couronne-6 ou l'hexaoxa-4, 7, 13, 16, 21, 24 diaza-1, 10 bicyclo [8,8,8] hexacosane.

10. Procédé selon l'une quelconque des revendications précédentes caractérisé en ce que lorsqu'on utilise le fluorure avec un agent complexant du cation la réaction a lieu en présence d'un ou de plusieurs solvants anhydres, inertes vis-à-vis du carbonate.

11. Procédé selon la revendication précédente caractérisé en ce que le solvant est choisi parmi les diéthers alkyliques d'alkylène glycol ou de polyoxyalkylène glycols, le benzonitrile ou le dioxanne.

12. Procédé selon l'une quelconque des revendications précédentes caractérisé en ce que la température de la réaction est comprise entre 30 et 70°.

13. Procédé selon l'une quelconque des revendications précédentes caractérisé en ce qu'on sépare le fluoroformiate et l'aldéhyde obtenus ou l'un des deux du milieu réactionnel par évaporation.

14. Procédé selon l'une quelconque des revendications précédentes caractérisé en ce que l'on stabilise le fluoroformiate purifié par addition de 1 à 25% de préférence 5 à 10% en poids par rapport à ce dernier, d'un carbonate alcalin ou alcalinoterreux.

## Patentansprüche

1. Verfahren zur Herstellung von Fluorformiaten der Formel

$$R^1\text{–O–}\underset{\underset{\displaystyle O}{\|}}{C}\text{–F}$$

in der $R^1$ ein ggf. substituierter, ggf. gesättigter aliphatischer, cycloaliphatischer oder polycyclischer Rest, ein ggf. substituierter arylaliphatischer Rest oder ein aromatischer Rest ist, gekennzeichnet durch Umsetzung eines Carbonats der Formel

$$R^1\text{–O–}\underset{\underset{\displaystyle O}{\|}}{C}\text{–O–}\underset{\underset{\displaystyle Cl}{|}}{CH}\text{–}R^2$$

in der $R^1$ die oben angegebene Bedeutung hat und $R^2$ ein Wasserstoffatom, einen ggf. gesättigten $C_{1-12}$-Alkyl- oder $C_{5-12}$-Cycloalkylrest, der ggf. mit einem oder mehreren Halogenatomen substituiert ist, oder einen ggf. mit einem oder mehreren Halogenatomen substituierten Arylrest bedeutet, mit einem Alkali- oder Erdalkalifluorid, einem Ammonium- oder quartären Ammoniumfluorid der Formel $NR^3R^4R^5R^6$, in der $R^3$, $R^4$, $R^5$ und $R^6$, gleich oder verschieden, ein Wasserstoffatom, einen $C_{1-12}$-Alkylrest oder $C_{1-12}$-Arylalkylrest bedeuten, oder mit einem anorganischen Fluorid, ausgewählt unter $KHF_2$, $NH_4HF_2$ oder $KSO_2F$, wobei das Fluorid entweder mit einem Komplexbildner für das Kation, ausgewählt unter Kryptaten, cyclischen oder geradkettigen Polyethern, oder mit einem aprotischen polaren Medium aktiviert wird, bei einer Reaktionstemperatur von 20 bis 120 °C, Abtrennen des erhaltenen Fluorformiats und/oder Aldehyds im Verlauf ihrer Bildung aus den Reaktionsmedien und Gewinnen des Fluorformiats.

2. Verfahren nach Anspruch 1, gekennzeichnet durch Verwendung einer Verbindung, in der $R^1$

ein tertiärer aliphatischer oder cycloaliphatischer Rest, ein polycycloaliphatischer Rest oder eine Benzylgruppe ist.

3. Verfahren nach Anspruch 2, gekennzeichnet durch Verwendung einer Verbindung, in der $R^1$ t-Butyl, t-Amyl oder 1-Adamantyl ist.

4. Verfahren nach einem der vorstehenden Ansprüche, gekennzeichnet durch Verwendung einer Verbindung, in der $R^2$ $-CCl_3$ ist.

5. Verfahren nach einem der vorstehenden Ansprüche, dadurch gekennzeichnet, dass das aprotische polare Medium unter Dimethylformamid, Dimethylsulfoxid, Hexamethylphosphortriamid, N-Methylpyrrolidon und Sulfolan ausgewählt wird.

6. Verfahren nach einem der vorstehenden Ansprüche, dadurch gekennzeichnet, dass das Fluorid in einer Menge von 1 bis 5 und vorzugsweise 1, 2 und 3 Äquivalenten, bezogen auf das Carbonat, verwendet wird.

7. Verfahren nach einem der vorstehenden Ansprüche, dadurch gekennzeichnet, dass der Komplexbildner in einer Menge von 1 bis 10 Mol-%, bezogen auf das Fluorid, zugegeben wird.

8. Verfahren nach einem der vorstehenden Ansprüche, gekennzeichnet durch Verwendung von Kaliumfluorid.

9. Verfahren nach einem der vorstehenden Ansprüche, dadurch gekennzeichnet, dass das Kaliumfluorid mit dem 18-Kronen-6-ether oder Hexaoxa-4, 7, 13, 16, 21, 24-diaza-1, 10-bicyclo[8,8,8] hexacosan zum Komplex verbunden wird.

10. Verfahren nach einem der vorstehenden Ansprüche, dadurch gekennzeichnet, dass bei Verwendung des Fluorids mit einem Komplexbildner für das Kation die Umsetzung in Gegenwart mindestens eines wasserfreien, gegenüber dem Carbonat inerten Lösungsmittel durchgeführt wird.

11. Verfahren nach Anspruch 10, dadurch gekennzeichnet, dass das Lösungsmittel unter Alkylenglykol- oder Polyoxyalkylenglykol-dialkylether, Benzonitril oder Dioxan ausgewählt wird.

12. Verfahren nach einem der vorstehenden Ansprüche, dadurch gekennzeichnet, dass die Umsetzung bei einer Temperatur von 30 bis 70 °C durchgeführt wird.

13. Verfahren nach einem der vorstehenden Ansprüche, dadurch gekennzeichnet, dass das erhaltene Fluorformiat und/oder der erhaltene Aldehyd aus dem Reaktionsmedium durch Verdampfung abgetrennt werden.

14. Verfahren nach einem der vorstehenden Ansprüche, dadurch gekennzeichnet, dass das gereinigte Fluorformiat durch Zugabe von 1 bis 25 Gew.-% und vorzugsweise 5 bis 10 Gew.-%, bezogen auf das Fluorformiat, eines Alkali- oder Erdalkalicarbonats stabilisiert wird.

## Claims

1. Process for the preparation of fluoroformates of formula

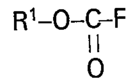

in which $R^1$ denotes a substituted or unsubstituted, saturated or unsaturated aliphatic, cycloaliphatic or polycyclic residue, a substituted or unsubstituted araliphatic residue or an aromatic residue, characterized in that a carbonate of formula

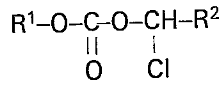

in which $R^1$ has the above meaning and $R^2$ denotes a hydrogen atom, a saturated or unsaturated $C_1$–$C_{12}$ alkyl or $C_5$–$C_{12}$ cycloalkyl residue, unsubstituted or substituted by one or more halogen atoms, or an aryl residue, unsubstituted or substituted by one or more halogen atoms, is reacted with a fluoride of an alkali or alkaline-earth metal, of ammonium or of quaternary ammonium $NR^3R^4R^5R^6$ in which $R^3$, $R^4$, $R^5$ and $R^6$, which are identical or different, denote a hydrogen atom or a $C_1$–$C_{12}$ alkyl or aralkyl group, or with an inorganic fluoride chosen from $KHF_2$, $NH_4HF_2$ and $KSO_2F$, the fluoride being activated either by a cationcomplexing agent chosen from cryptates and cyclic or linear polyethers, or by a polar aprotic medium, at a reaction temperature of between 20 and 120 °C, that the fluoroformate or aldehyde obtained, or both, are separated from the reaction medium as they are formed, and that the fluoroformate is recovered.

2. Process according to claim 1, characterized in that $R^1$ denotes a tertiary aliphatic or cycloaliphatic group, a polycycloaliphatic group or a benzyl group.

3. Process according to the preceding claim, characterzed in that $R^1$ denotes the t-butyl, t-amyl or 1-adamantyl group.

4. Process according to any one of the preceding claims, characterized in that $R^2$ is the $-CCl_3$ radical.

5. Process according to any one of the preceding claims, characterized in that the polar aprotic medium is chosen from dimethylformamide, dimethyl sulphoxide, hexamethylphosphorotriamide, N-methylpyrrolidone and sulpholane.

6. Process according to any one of the preceding claims, characterized in that the fluoride is employed in a quantity of between 1 and 5 equivalents, preferably 1, 2 and 3 equivalents, relative to the carbonate.

7. Process according to any one of the preceding claims, characterized in that the complexing agent is added in a proportion of 1 to 10 mol% relative to the fluoride.

8. Process according to any one of the preceding claims, characterized in that the fluoride is potassium fluoride.

9. Process according to the preceding claim, characterized in that the potassium fluoride is complexed with 18-crown-6 or 4, 7, 13, 16, 21, 24-hexaoxa-1, 10-diazabicyclo [8,8,8,] hexacosane.

10. Process according to any one of the preceding claims, characterized in that, when the fluoride is employed with a cation-complexing agent, the reaction takes place in the presence of one or more anhydrous solvents which are inert towards the carbonate.

11. Process according to the preceding claim, characterized in that the solvent is chosen from alkylene glycol or polyoxyalkylene glycol dialkyl ethers, benzonitrile or dioxane.

12. Process according to any one of the preceding claims, characterized in that the reaction temperature is between 30 and 70°.

13. Process according to any one of the preceding claims, characterized by the separation of the fluoroformate and the aldehyde which are obtained, or either of the two, from the reaction medium by evaporation.

14. Process according to any one of the preceding claims, characterized in that the purified fluoroformate is stabilized by the addition of 1 to 25%, preferably 5 to 10%, by weight relative to the latter, of an alkali or alkaline-earth metal carbonate.